Europäisches Patentamt

European Patent Office

Office européen des brevets

(19)

(11) Numéro de publication : **0 343 707 B1**

# FASCICULE DE BREVET EUROPEEN

(12)

(45) Date de publication du fascicule du brevet :
**19.08.92 Bulletin 92/34**

(51) Int. Cl.⁵ : **B01J 23/58,** C07C 17/00,
C07C 21/18

(21) Numéro de dépôt : **89201198.2**

(22) Date de dépôt : **12.05.89**

(54) **Procédé pour l'hydrogénation de chlorofluoralcènes.**

(30) Priorité : **24.05.88 FR 8806991**

(43) Date de publication de la demande :
**29.11.89 Bulletin 89/48**

(45) Mention de la délivrance du brevet :
**19.08.92 Bulletin 92/34**

(84) Etats contractants désignés :
**AT BE CH DE ES FR GB GR IT LI LU NL SE**

(56) Documents cités :
**EP-A- 0 053 657
EP-A- 0 257 561
FR-A- 2 583 039
US-A- 2 476 920
US-A- 3 505 417
US-A- 3 846 281**

(73) Titulaire : **SOLVAY & Cie (Société Anonyme)
Rue du Prince Albert, 33
B-1050 Bruxelles (BE)**

(72) Inventeur : **Lerot, Luc
Avenue du Centaure, 31
B-1200 Bruxelles (BE)**
Inventeur : **Costa, Jean-Louis
Place Peter Benoît, 8
B-1120 Bruxelles (BE)**
Inventeur : **Wilmet, Vincent
Scavée du Biéreau 13/101
B-1348 Louvain-la-Neuve (BE)**
Inventeur : **Pirotton, Joseph
Rue Desiré Desmet, 28
B-1030 Bruxelles (BE)**

EP 0 343 707 B1

## Description

L'invention concerne un procédé pour l'hydrogénation de chlorofluoralcènes en fluoralcènes et plus particulièrement l'hydrogénation de chlorotrifluoréthylène en trifluoréthylène.

L'hydrogénation du chlorotrifluoréthylène en trifluoréthylène à l'intervention de catalyseurs comprenant d'une part un support tel que l'alumine et d'autre part un métal du groupe VIII de la table périodique des éléments est une réaction connue depuis longtemps (brevet US 2697124).

Ces catalyseurs ont subi des améliorations multiples et ont conduit à des procédés tels que notamment décrits dans le brevet européen 53657 qui concerne un procédé d'hydrogénation de chlorotrifluoréthylène en trifluoréthylène à l'intervention de catalyseurs constitués d'un métal du groupe du platine déposé sur un support particulier tel qu'un sel mixte de fluorure de sodium magnésium ou de fluorure de potassium magnésium; ce catalyseur peut être réactivé à température élevée, telle que 400 à 600°C, par un gaz contenant de l'oxygène.

Toutefois, tous les procédés de synthèse catalytique connus à ce jour ont une activité catalytique relativement faible et sont accompagnés de réactions secondaires et/ou une désactivation rapide des catalyseurs, ce qui compromet l'efficacité de ces procédés.

L'invention, par contre, concerne un procédé de synthèse catalytique qui ne présente plus ces inconvénients. On a en effet trouvé des compositions catalytiques qui permettent d'hydrogéner les chlorofluoralcènes avec une sélectivité et un taux de transformation, c'est-à-dire un rendement, qui n'ont jamais été atteints industriellement, qui en outre présentent l'avantage d'être stables et de se désactiver beaucoup plus lentement que les compositions catalytiques connues et d'être régénérables à température modérée.

L'invention concerne à cet effet un procédé pour l'hydrogénation de chlorofluoralcènes en fluoralcènes en présence d'une composition catalytique comprenant un support poreux sur lequel sont déposés un métal du groupe VIII de la table périodique des éléments et un ou plusieurs composés choisis parmi les sels d'un métal alcalin ou alcalino-terreux.

Les sels d'un métal alcalin ou alcalino-terreux utilisés sont des sels organiques ou inorganiques de ces métaux. Comme sels organiques, on utilise généralement des carboxylates, des alcoolates, des acétylacétonates, dont la chaîne alkyle contient de 1 à 10 atomes de carbone habituellement. Comme sels inorganiques, on utilise généralement des halogénures, des hydroxydes ou des nitrates et, plus particulièrement, les halogénures ou les hydroxydes d'un métal alcalin ou alcalino-terreux, tels que les chlorures, fluorures ou hydroxydes de sodium, potassium, césium, lithium, baryum, calcium, rubidium. De manière avantageuse, on choisit les chlorures, fluorures ou hydroxydes de sodium, potassium, césium ou baryum, tels que le chlorure de césium, le chlorure de potassium, le chlorure de baryum, le fluorure de césium et l'hydroxyde de césium. De manière préférée, on utilise le chlorure de césium, le chlorure de potassium ou le chlorure de baryum.

La composition catalytique peut comprendre un ou plusieurs composés choisis parmi les sels d'un métal alcalin ou alcalinoterreux. De bons résultats ont été obtenus avec un ou deux de ces composés. On utilise de préférence une composition binaire choisie parmi les chlorures de césium, potassium ou baryum. Particulièrement préférées est la composition contenant simultanément du chlorure de baryum et du chlorure de césium.

La composition catalytique comprend de 1 à 25 % en poids de métal alcalin ou alcalino-terreux par rapport au poids total de la composition catalytique. De préférence, elle comprend de 5 à 20 % en poids de métal alcalin ou alcalino-terreux par rapport au poids total de la composition catalytique. Lorsqu'on utilise plusieurs composés, les proportions entre chaque composé peuvent varier entre de larges limites. De bons résultats ont été obtenus avec le chlorure de baryum et le chlorure de césium mis en oeuvre dans des rapports en baryum et césium compris entre 2:1 et 1:2.

Comme métal du groupe VIII de la table périodique des éléments, on utilise habituellement le palladium, le platine, le rhodium, le ruthénium, le cobalt ou le nickel et, de préférence, le palladium ou le platine.

La composition catalytique comprend habituellement de 0,05 à 10 % en poids de métal du groupe VIII par rapport au poids total de la composition catalytique et de préférence de 0,1 à 5 %. De bons résultats ont été obtenus lorsque le rapport pondéral entre le métal alcalin ou alcalino- terreux et le métal du groupe VIII est compris entre 0,1 et 15 et, plus particulièrement lorsque ce rapport est compris entre 2 et 6.

Habituellement, on utilise comme support de la composition catalytique un support poreux, tel que ceux couramment employés dans les compositions catalytiques antérieures utilisées dans ce type de réaction d'hydrogénation. Généralement, on utilise un support à base de carbone, charbon actif, alumine, fluorure d'alcali magnésium, silice, alumine fluorée, oxyde de titane, oxyde de magnésium, oxyde de zirconium, fluorure de zinc ou des hydroxydes de ces métaux. De bons résultats ont été obtenus avec l'alumine, la silice et le mélange d'alumine et de silice, ainsi qu'avec l'oxyde de titane, l'oxyde de zirconium et l'oxyde et/ou l'hydroxyde de magnésium.

Le volume poreux du support peut varier entre de larges limites, il est généralement compris entre 0,1 et 5 cm³/g et habituellement entre 0,4 et 2 cm³/g.

La surface spécifique du support peut varier entre de larges limites, elle est généralement comprise entre 5 et 1000 m²/g, et habituellement entre 10 et 550 m²/g.

La composition catalytique peut être obtenue selon divers procédés.

A cet effet, la composition catalytique peut être obtenue par imprégnation du support avec des solutions contenant le métal du groupe VIII de la table périodique des éléments et un ou plusieurs composés choisis parmi les sels d'un métal alcalin ou alcalino-terreux. Cette imprégnation peut être réalisée par n'importe quelle méthode, telle que notamment par la technique dite du "volume poreux" (imprégnation dite "sèche") ou par la technique du "volume excédentaire" (imprégnation par voie dite "humide"); ces méthodes sont décrites dans le livre Charles N. Satterfield "Heterogeneous catalysis in practice", 1980 Mc Graw-Hill Book Company, New York, en particulier pages 82 et 83.

Le métal du groupe VIII de la table périodique des éléments est habituellement introduit dans la composition catalytique sous la forme d'un sel de ce métal. Pour ce faire, on met généralement en oeuvre un chlorure ou un complexe ammoniacal du métal du groupe VIII.

Les solutions d'imprégnation peuvent être aqueuses ou organiques, de préférence on met en oeuvre une solution aqueuse ou alcoolique.

L'imprégnation peut être réalisée d'abord avec une solution contenant le métal du groupe VIII de la table périodique des éléments, ou d'abord avec une solution contenant un ou plusieurs composés choisis parmi les sels d'un métal alcalin ou alcalinoterreux, ou simultanément avec les deux solutions.

Un mode d'obtention de la composition catalytique ayant donné de bons résultats consiste à imprégner le support lors d'une première étape avec une solution aqueuse contenant un ou plusieurs composés choisis parmi les sels d'un métal alcalin ou alcalino-terreux, puis, après séchage, lors d'une deuxième étape avec une solution aqueuse contenant un sel du métal du groupe VIII de la table périodique des éléments, sel soluble dans l'eau, tel que notamment un chlorure ou un complexe ammoniacal. De bons résultats ont été obtenus avec un chlorure. Ces imprégnations se font généralement à température ambiante avec une solution aqueuse renfermant les quantités désirées de sels d'un métal alcalin ou alcalino-terreux, puis de sel du métal du groupe VIII. Le séchage entre les deux imprégnations a lieu à 350°C pendant 2 heures. Ce support imprégné est ensuite séché à 120°C, puis introduit dans le réacteur d'hydrogénation proprement dit. La composition catalytique ainsi obtenue peut être mise en oeuvre telle quelle ou peut être préalablement réduite soit par de l'hydrogène, soit par un mélange d'hydrogène avec un gaz inerte tel que l'hélium. La température à laquelle est effectuée cette réduction est généralement comprise entre 100 et 500°C, de bons résultats ont été obtenus avec une température de réduction comprise entre 150 et 250°C. La pression à laquelle est effectuée cette réduction est généralement comprise entre 1 et 5 bars.

La composition catalytique peut être mise en oeuvre dans tout procédé d'hydrogénation, tel que notamment les procédés réalisés avec un catalyseur disposé en lit fixe ou en lit fluidisé.

La composition catalytique peut être mise en oeuvre pour la synthèse de n'importe quel fluoralcène à partir du chlorofluoralcène correspondant. De bons résultats ont été obtenus pour la synthèse de trifluoréthylène à partir de chlorotrifluoréthylène.

Plus particulièrement, l'invention concerne un procédé d'hydrogénation de chlorotrifluoréthylène en trifluoréthylène dans lequel la réaction est catalysée par une composition catalytique comprenant un support poreux sur lequel sont déposés un métal du groupe VIII de la table périodique des éléments et un ou plusieurs composés choisis parmi les sels d'un métal alcalin ou alcalino-terreux.

La température à laquelle s'effectue la réaction d'hydrogénation se situe habituellement entre 80 et 600°C. De préférence, cette température est comprise entre 120 et 400°C. De bons résultats ont été obtenus avec une température réactionnelle située aux environs de 200-300°C.

La pression à laquelle est effectuée la réaction d'hydrogénation n'est pas critique en elle-même. Habituellement, on opère avec des pressions comprises entre 1 et 10 bars et, de préférence, avec des pressions comprises entre 2 et 5 bars.

Le rapport volumique entre le chlorotrifluoréthylène et l'hydrogène mis en oeuvre est généralement compris entre 0,05 et 4. De préférence, ce rapport est compris entre 0,1 et 2,5. De bons résultats ont été obtenus avec un rapport situé aux environs de 1.

Le temps de contact moyen est généralement compris entre 2 et 16 s, habituellement ce temps est compris entre 3 et 10 s. De bons résultats ont été obtenus avec un temps de contact compris entre 4 et 8 s.

Le procédé selon l'invention permet d'obtenir une sélectivité supérieure à 90 % et la plupart du temps supérieure à 95 %, la production de sous-produits est faible, la plupart du temps inférieure à 5 %. Le taux de transformation de chlorotrifluoréthylène est élevé, supérieur à 40 %.

Après utilisation de la composition catalytique, on observe que la régénération de la composition catalytique est aisée et peut se réaliser in situ dans le réacteur d'hydrogénation. Un mode de régénération ayant

3

donné de bons résultats consiste à régénérer la composition catalytique sous courant d'air, puis sous courant d'hydrogène. Les performances des compositions catalytiques après la régénération sont très proches de celles observées avec une composition catalytique fraîche. Cette régénération des compositions catalytiques est généralement réalisée à température modérée, c'est-à-dire à des températures comprises entre 100 et 400°C et de préférence 200 et 300°C.

L'invention se trouve plus amplement illustrée par les exemples suivants.

Exemple 1 :

a) Préparation de la composition catalytique

On introduit dans une ampoule à imprégnation cylindrique de 40 cm³ 10 g de silice ayant les caractéristiques suivantes :
– surface spécifique B.E.T. de 250 m²/g
– volume poreux environ 0,8 cm³/g.

On chauffe l'ampoule sous vide (3 mm Hg) durant 2 heures à 350°C dans un four cylindrique en vue de dégazer la silice.

Après refroidissement sous vide, on imprègne la silice à température ambiante sous vide dans un volume de 8,8 cm³ d'une solution aqueuse contenant 1 g de chlorure de césium et 1,25 g de chlorure de baryum, le volume de cette solution correspond au volume poreux du support augmenté de 10 %.

On laisse reposer 1 heure sous vide statique, puis une nuit à pression atmosphérique à température ambiante.

Ensuite, on sèche la silice ainsi imprégnée à 350°C sous vide (1 à $6.10^2$ Pa) durant 2 heures.

On imprègne ensuite cette silice à température ambiante sous vide (1 à $6.10^2$ Pa) dans un volume de 8,8 cm³ d'une solution contenant 0,17 g de chlorure de palladium dans de l'eau acidifiée par 4 % en volume d'acide chlorhydrique concentré, le volume de cette solution correspond au volume poreux du support augmenté de 10 %.

On laisse reposer 1 heure sous vide, puis une nuit à pression atmosphérique à température ambiante.

Puis, on sèche 3 heures à 120°C à pression atmosphérique.

La composition catalytique ainsi obtenue comprend 0,8 % en poids de palladium, 6,6 % en poids de baryum et 6,4 % en poids de césium par rapport au poids total de la composition catalytique.

2 cm³ de cette composition catalytique sont introduits dans un réacteur d'hydrogénation constitué d'un tube métallique en inox long de 520 mm et d'un diamètre intérieur de 7,7 mm; puis, la composition catalytique est conditionnée 2 heures à 500°C sous 3 bars au moyen d'un mélange d'hydrogène et d'hélium dans un rapport volumique 1/9 à un débit de 40 cm³/mn.

b) Hydrogénation du chlorotrifluoréthylène

On alimente le réacteur à raison de 0,05 mole par heure de chlorotrifluoréthylène et 0,05 mole par heure d'hydrogène à 240°C sous 3 bars. Le temps de contact moyen est évalué à 5,3 s.

Après 4 heures de fonctionnement, le taux de transformation du chlorotrifluoréthylène en trifluoréthylène est de 60 %, la sélectivité est supérieure à 96 % en trifluoréthylène.

Après 100 heures de fonctionnement, le taux de transformation est de 55 % avec une sélectivité de 94 %.

c) Régénération de la composition catalytique

Après usage, on régénère la composition catalytique in situ dans le réacteur d'hydrogénation.

Pour ce faire, on introduit dans le réacteur un courant d'air durant 2 heures, puis un courant d'hydrogène durant 2 heures à 240°C.

Les performances de la composition catalytique après cette régénération sont comparables à celles observées avec une composition catalytique fraîche.

Exemples 2, 3 et 4 :

Hydrogénation du chlorotrifluoréthylène

On charge le réacteur, constitué d'un tube métallique en inox long de 520 mm et d'un diamètre intérieur de 7,7 mm, avec 2 cm³ d'une composition catalytique telle que décrite à l'exemple 1.

On réduit la composition catalytique à 240°C pendant 2 heures par un débit de 0,01 mole d'hydrogène par heure dilué 10 fois à l'hélium sous 3 bars.

On alimente ce réacteur en chlorotrifluoréthylène et en hydrogène à 240°C sous 3 bars avec les quantités reprises au tableau 1 ci-après. Le temps de contact moyen est évalué à 5,3 s.

Les résultats après 4 heures de fonctionnement (le taux de transformation du chlorotrifluoréthylène en trifluoréthylène et la sélectivité) sont également rassemblés dans le tableau 1.

## Tableau 1

| EXEMPLES | ALIMENTATION DU REACTEUR EN MOLE PAR HEURE DE | | TAUX DE TRANSFORMATION DU CHLOROTRIFLUOR-ETHYLENE EN TRIFLUORETHYLENE % VOLUMIQUE | SELECTIVITE EN TRIFLUOR-ETHYLENE % VOLUMIQUE |
|---|---|---|---|---|
| | CHLOROTRI-FLUOR-ETHYLENE | HYDROGENE | | |
| 2 | 0,05 | 0,05 | 54 | 95 |
| 3 | 0,015 | 0,08 | 80 | 93 |
| 4 | 0,050 | 0,025 | 46 | 97 |

Exemple 5 :

On charge le réacteur décrit dans l'exemple 1 avec une composition catalytique comprenant un support poreux constitué d'alumine de surface spécifique 350 m$^2$/g et de volume poreux 1,7 cm$^3$/g et imprégné de 19 % en poids de BaCl$_2$ (13,6 % de baryum par rapport au poids total de la composition catalytique) et de 4,3 % en poids de PdCl$_2$ (2,8 % en poids de palladium par rapport au poids total de la composition catalytique) selon la méthode de préparation décrite à l'exemple 1.

On réduit la composition catalytique à 500°C pendant 2 heures par un débit de 0,01 mole d'hydrogène par heure dilué 10 fois à l'hélium sous 3 bars.

On alimente le réacteur à raison de 0,05 mole par heure de chlorotrifluoréthylène et 0,05 mole par heure d'hydrogène à 240°C sous 3 bars. Le temps de contact moyen est évalué à 3 s.

Le taux de transformation du chlorotrifluoréthylène est de 86 % après 1 h de fonctionnement, de 80 % après 24 h et de 77 % après 70 h.

La sélectivité s'élève à 95 % en trifluoréthylène.

Exemple 6 :

On charge le réacteur décrit dans l'exemple 1 avec une composition catalytique comprenant un support poreux constitué de silice, ayant une surface spécifique de 250 m$^2$/g et un volume poreux de 1,7 cm$^3$/g, et imprégné de 16 % en poids de CsCl et de 4,5 % en poids de PdCl$_2$ selon la méthode de préparation décrite à l'exemple 1.

On réduit la composition catalytique à 500°C pendant 2 heures par un débit de 0,01 mole d'hydrogène par heure dilué 10 fois à l'hélium sous 1 bar.

On alimente le réacteur à raison de 0,05 mole par heure de chlorotrifluoréthylène et 0,05 mole par heure d'hydrogène à 240°C sous 1 bar. Le temps de contact moyen est évalué à 1,7 s.

Après 16 heures de fonctionnement, le taux de transformation du chlorotrifluoréthylène est de 54 %.

La sélectivité s'élève à 95 % en trifluoréthylène.

Exemple 7 :

Une composition catalytique est préparée en suivant le protocole décrit à l'exemple 1a.

Le support est constitué de silice telle que celle décrite à l'exemple 1.

On met en oeuvre 6,2 % en poids de baryum, 5,2 % en poids de césium et 0,83 % en poids de palladium calculé par rapport au poids total de la composition catalytique réduite, ces métaux ont été mis en oeuvre sous forme de chlorure.

La composition catalytique est réduite en dehors du réacteur à 150°C sous 3 bars au moyen d'un mélange d'hydrogène et d'hélium dans un rapport volumique 1/9 à un débit de 40 cm$^3$/mn.

La composition catalytique est ensuite introduite dans le réacteur d'hydrogénation identique à celui décrit à l'exemple 1.

On alimente le réacteur à raison de 0,05 mole par heure de chlorotrifluoréthylène et 0,05 mole par heure d'hydrogène à 280°C sous 3 bars. Le temps de contact moyen est évalué à 4,9 s.

Après 4 heures de fonctionnement, le taux de transformation de chlorotrifluoréthylène en trifluoréthylène est de 60 %, la sélectivité est de 94 % en trifluoréthylène.

Après 16 heures de fonctionnement, le taux de transformation est de 66 %, la sélectivité est de 94 %.

Exemple 8 :

En suivant le protocole décrit à l'exemple 1, on prépare une composition catalytique comprenant un support poreux constitué de silice, ayant une surface spécifique de 250 m$^2$/g et un volume poreux de 1,7 cm$^3$/g, et imprégné de 13,5 % en poids de CsCl (10,6 % en poids de césium par rapport au poids total de la composition catalytique réduite) et de 1,4 % en poids de PdCl$_2$ (0,85 % en poids de palladium par rapport au poids total de la composition catalytique réduite).

On réduit la composition catalytique à 150°C en dehors du réacteur pendant 2 heures par un débit de 0,01 mole d'hydrogène par heure dilué 10 fois à l'hélium sous 1 bar.

On introduit cette composition catalytique dans un réacteur, identique à celui décrit à l'exemple 1.

On alimente le réacteur à raison de 0,05 mole par heure de chlorotrifluoréthylène et 0,05 mole par heure d'hydrogène à 240°C sous 3 bars. Le temps de contact moyen est évalué à 5,3 s.

Après 4 heures de fonctionnement, le taux de transformation du chlorotrifluoréthylène est de 41 %.

La sélectivité s'élève à 95 % en trifluoréthylène.

Après 16 heures de fonctionnement, le taux de transformation du chlorotrifluoréthylène est de 45 % et la sélectivité de 96 %.

Exemple 9

a) Préparation de la composition catalytique

On introduit dans une ampoule à imprégnation cylindrique de 40 cm$^3$ 10 g d'oxyde de magnésium ayant les caractéristiques suivantes :
– surface spécifique B.E.T. de 23 m$^2$/g
– volume poreux environ 0,3 cm$^3$/g,
et préalablement imbibés par 2 cm$^3$ d'eau, laissés au repos 16 heures puis séchés sous vide (100 à 200 Pa).

On chauffe l'ampoule sous vide (3 mm Hg) durant 2 heures à 350°C dans un four cylindrique en vue de dégazer l'oxyde de magnésium.

Après refroidissement sous vide, on imprègne l'oxyde de magnésium à température ambiante sous vide dans un volume de 2 cm$^3$ d'une solution aqueuse contenant 0,52 g de chlorure de césium.

On laisse reposer 1 heure sous vide statique, puis une nuit à pression atmosphérique à température ambiante.

Ensuite, on sèche l'oxyde de magnésium ainsi imprégné à 90°C sous vide (1 à 6.10$^2$ Pa) durant 2 heures.

On imprègne ensuite cet oxyde de magnésium à température ambiante sous vide (1 à 6.10$^2$ Pa) dans un volume de 2 cm$^3$ d'une solution contenant 0,08 g de chlorure de palladium dans de l'eau acidifiée par 10 % en volume d'acide chlorhydrique concentré.

On laisse reposer 1 heure sous vide, puis une nuit à pression atmosphérique à température ambiante.

Puis, on sèche 2 heures à 90°C sous vide (1 à 6.10$^2$ Pa).

La composition catalytique ainsi obtenue comprend 0,45 % en poids de palladium et 4 % en poids de césium par rapport au poids total de la composition catalytique.

2 cm$^3$ de cette composition catalytique sont introduits dans un réacteur d'hydrogénation constitué d'un tube métallique en inox long de 520 mm et d'un diamètre intérieur de 7,7 mm; puis, la composition catalytique est conditionnée 2 heures à 500°C sous 3 bars au moyen d'un mélange d'hydrogène et d'hélium dans un rapport volumique 1/9 à un débit de 40 cm³/mn.

b) Hydrogénation du chlorotrifluoréthylène

On alimente le réacteur à raison de 0,05 mole par heure de chlorotrifluoréthylène et 0,05 mole par heure d'hydrogène à 240°C sous 3 bars. Le temps de contact moyen est évalué à 5,3 s.

Après 10 heures de fonctionnement, le taux de transformation du chlorotrifluoréthylène en trifluoréthylène est de 64 %, la sélectivité est supérieure à 94 % en trifluoréthylène.

Après 70 heures de fonctionnement, le taux de transformation et la sélectivité sont inchangés.

## Revendications

1. Procédé pour l'hydrogénation de chlorofluoralcènes en fluoralcènes en présence d'une composition catalytique comprenant un support poreux et un métal du groupe VIII de la table périodique des éléments, caractérisé en ce que cette composition catalytique comprend en outre un ou plusieurs composés choisis parmi les sels d'un métal alcalin ou alcalino-terreux.

2. Procédé selon la revendication 1, caractérisé en ce que les sels d'un métal alcalin ou alcalino-terreux utilisés sont des halogénures ou des hydroxydes de ces métaux.

3. Procédé selon la revendication 2, caractérisé en ce que les halogénures ou les hydroxydes d'un métal alcalin ou alcalinoterreux utilisés sont choisis parmi les chlorures, fluorures ou hydroxydes de potassium, césium ou baryum.

4. Procécé selon la revendication 2 ou 3, caractérisé en ce que la composition catalytique comprend deux composés choisis parmi les halogénures ou les hydroxydes d'un métal alcalin ou alcalino-terreux.

5. Procédé selon les revendications 1, 2, 3 ou 4, caractérisé en ce que la composition catalytique comprend de 1 à 25 % en poids de métal alcalin ou alcalino-terreux.

6. Procédé selon l'une quelconque des revendications précédentes, caractérisé en ce que le métal du groupe VIII de la table périodique des éléments utilisé est choisi parmi le palladium ou le platine.

7. Procédé selon l'une quelconque des revendications précédentes, caractérisé en ce que la composition catalytique comprend de 0,05 à 10 % en poids de métal du groupe VIII.

8. Procédé selon l'une quelconque des revendications précédentes, caractérisé en ce que le support utilisé est à base d'alumine, de silice, d'un mélange d'alumine et de silice, d'oxyde de titane ou d'oxyde de zirconium.

9. Procédé selon l'une quelconque des revendications précédentes, caractérisé en ce que le chlorofluoralcène est le chlorotrifluoréthylène.

## Patentansprüche

1. Hydrierungsverfahren von Chlorfluoroalkenen in Fluoroalkene in Anwesenheit einer katalytischen Zusammensetzung, die einen porösen Träger und ein Metall der Gruppe VIII der Tabelle des periodischen Systems umfaßt, dadurch gekennzeichnet, daß diese katalytische Zusammensetzung außerdem eine oder mehrere Verbindungen, ausgewählt unter den Salzen eines Alkali- oder Erdalkalimetalls, umfaßt.

2. Verfahren nach Anspruch 1, dadurch gekennzeichnet, daß die verwendeten Salze eines Alkali- öder Erdalkalimetalls Halogenide oder Hydroxyde dieser Metalle sind.

3. Verfahren nach Anspruch 2, dadurch gekennzeichnet, daß die verwendeten Halogenide oder Hydroxyde eines Alkalioder Erdalkalimetalls ausgewählt sind unter den Chloriden, Fluoriden oder Hydroxyden von Kalium, Cäsium oder Barium.

4. Verfahren nach Anspruch 2 oder 3, dadurch gekennzeichnet; daß die katalytische Zusammensetzung zwei Verbindungen umfaßt, ausgewählt unter den Halogeniden oder Hydroxyden eines Alkali- oder Erdalkalimetalls.

5. Verfahren nach den Ansprüchen 1, 2, 3 oder 4, dadurch gekennzeichnet, daß die katalytische Zusammensetzung 1 bis 25 Gew.-% Alkali- oder Erdalkalimetall umfaßt.

6. Verfahren nach einem der vorhergehenden Ansprüche, dadurch gekennzeichnet, daß des verwendete Metall der Gruppe VIII der Tabelle des periodischen Systems ausgewählt ist unter Palladium oder Platin.

7. Verfahren nach einem der vorhergehenden Ansprüche, dadurch gekennzeichnet, daß die katalytische Zusammensetzung 0,05 bis 10 Gew.-% des Metalls der Gruppe VIII umfaßt.

8. Verfahren nach einem der vorhergehenden Ansprüche, dadurch gekennzeichnet, daß der verwendete Träger auf der Basis von Aluminiumoxyd, Kieselerde, einer Mischung von Aluminiumoxyd und Kieselerde, Titanoxyd oder Zirkoniumoxyd ist.

9. Verfahren nach einem der vorhergehenden Ansprüche, dadurch gekennzeichnet, daß das Chlorfluoroalken Chlortrifluorethylen ist.

## Claims

1. Process for the hydrogenation of chlorofluoroalkenes to fluoroalkenes in the presence of a catalytic composition comprising a porous carrier and a metal of group VIII of the Periodic Table of the elements, characterised in that this catalytic composition furthermore comprises one or more compounds chosen from the salts of an alkali metal or alkaline-earth metal.

2. Process according to Claim 1, characterised in that the salts of an alkali metal or alkaline-earth metal which are employed are halides or hydroxides of these metals.

3. Process according to Claim 2, characterised in that the halides or the hydroxides of an alkali metal or alkaline-earth metal which are employed are chosen from potassium, caesium or barium chlorides, fluorides or hydroxides.

4. Process according to Claim 2 or 3, characterised in that the catalytic composition comprises two compounds chosen from the halides or the hydroxides of an alkali metal or alkaline-earth metal.

5. Process according to Claims 1, 2, 3 or 4, characterised in that the catalytic composition comprises from 1 to 25% by weight of alkali metal or alkaline-earth metal.

6. Process according to any one of the preceding claims, characterised in that the metal of group VIII of the Periodic table of the elements which is employed is chosen from palladium or platinum.

7. Process according to any one of the preceding claims, characterised in that the catalytic composition comprises from 0.05 to 10% by weight of metal of group VIII.

8. Process according to any one of the preceding claims, characterised in that the carrier used is based on alumina, silica, a mixture of alumina and silica, titanium oxide or zirconium oxide.

9. Process according to any one of the preceding claims, characterised in that the chlorofluoroalkene is chlorotrifluoroethylene.